(19) Europäisches Patentamt
European Patent Office
Office européen des brevets

(11) **EP 3 112 824 B1**

(12) **EUROPÄISCHE PATENTSCHRIFT**

(45) Veröffentlichungstag und Bekanntmachung des Hinweises auf die Patenterteilung:
**30.09.2020  Patentblatt 2020/40**

(51) Int Cl.:
*G01G 9/00* (2006.01)      *G01G 17/00* (2006.01)
*G01N 33/15* (2006.01)      *G01N 22/04* (2006.01)

(21) Anmeldenummer: **16173923.0**

(22) Anmeldetag: **10.06.2016**

(54) **VORRICHTUNG UND VERFAHREN ZUR ERMITTLUNG EINER FÜR EIN OBJEKT CHARAKTERISTISCHEN GRÖSSE**

DEVICE AND METHOD FOR DETERMINING A CHARACTERISTIC VALUE FOR AN OBJECT

DISPOSITIF ET PROCEDE DE DETERMINATION D'UNE TAILLE CARACTERISTIQUE D'UN OBJET

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.06.2015  DE 102015110491**

(43) Veröffentlichungstag der Anmeldung:
**04.01.2017  Patentblatt 2017/01**

(73) Patentinhaber: **Work Microwave GmbH**
**83607 Holzkirchen (DE)**

(72) Erfinder:
• **Prokoph, Günther**
**83624 Otterfing (DE)**
• **Römhild, Hinrich**
**83607 Holzkirchen (DE)**
• **Rösler, Steffen**
**83607 Holzkirchen (DE)**

(74) Vertreter: **2SPL Patentanwälte PartG mbB**
**Postfach 15 17 23**
**80050 München (DE)**

(56) Entgegenhaltungen:
EP-A1- 1 703 275      WO-A1-2008/141833
US-A- 6 163 158

**Beschreibung**

Ausführungsbeispiele der vorliegenden Erfindung betreffen allgemein Vorrichtungen und Verfahren zur Ermittlung von für ein Objekt charakteristischen Größen, wie beispielsweise eine Masse und/oder einen Feuchtigkeitsgehalt des Objekts.

Derartige Verfahren und/oder Vorrichtungen können beispielsweise in der industriellen Massenproduktion von Medikamenten eingesetzt werden. In diesem Fall dienen portionierte Medikamente, beispielsweise in Form von Tabletten, als Objekt. Selbstverständlich sind auch weitere Einsatzgebiete möglich, bei denen es auf hohe Geschwindigkeiten bei der Bestimmung von für ein Objekt charakteristischen Größen ankommt.

Tabletten bleiben auf absehbare Zeit die wichtigste Darreichungsform von Medikamenten. Die Anforderungen an deren Produktion steigen zusehends deutlich an: Erhöhter Wettbewerb und Kostendruck haben das Thema Effizienz in den Vordergrund gerückt. Die Produktion neuartiger, komplexer Präparate muss entsprechend qualitätsorientiert und effizient erfolgen - und das bei einer steigenden Zahl von Produktionswechseln. High-Speed-Tablettierung mit einem Ausstoß von mehr als 1,6 Millionen Tabletten pro Stunde sind derzeit die Maßstäbe für Produktivität in der Tablettierung. Gleichzeitig müssen Pharmaproduzenten an allen Standorten hohe Qualitätsstandards sicherstellen, um die Richtlinien der Kontrollbehörden zu erfüllen. Dies macht eine 100% Produktkontrolle erforderlich. Daraus resultieren als Anforderung an Messsysteme sehr hohe Messgeschwindigkeiten, bei gleichzeitig möglichst geringem oder besser keinem Einfluss auf den Massestromtransport.

Bisherige Produktkontrollen erfolgen oft durch mechanische Wiegesysteme. Ein mechanischer Wiegevorgang benötigt allerdings eine gewisse Zeit, um einen Messwert einschwingen zulassen. Diese Zeit kann nicht beliebig verkürzt werden. Man kann dem Problem z.B. durch eine große Anzahl von parallel angeordneten Einrichtungen für die Verwiegung teilweise begegnen. Dadurch werden das Messsystem und die Tablettenzuführung aber sehr umfangreich, teuer und störanfällig. Die einzelnen Tabletten müssen zudem aus dem kontinuierlichen Transportstrom mechanisch entnommen und auf einem Wiegegutaufnehmer positioniert werden. Außerdem ist durch die empfindliche mechanische Führung bei derartigen Waagen bei Änderung des Formates der zu wiegenden Wirkstoffeinheiten ein erheblicher mechanischer Umbau von Produktführungen erforderlich.

Es gibt verschiedene Ansätze, um die obigen Nachteile, die vor allem durch die Trägheit der Wiegetechnik hervorgerufen werden, durch kapazitive Messtechniken zu überwinden. Derartige Ansätze werden beispielsweise in US 4,223,751 A, US 5,602,485 A oder DE-OS 29 39 406 beschrieben. Es kann mit solchen Methoden bei mit hoher Geschwindigkeit durch einen Kondensator bewegten Wirkstoffeinheiten ein masseproportionales elektrisches Signal nur aber dann bestimmt werden, wenn der Feuchtegehalt der Wirkstoffeinheiten und des Füllmaterials exakt konstant bleibt. Durch leichte Fluktuationen des Wassergehaltes des Wirkstoffs in den Wirkstoffeinheiten wird infolge der großen Dielektrizitätskonstante des Wassers eine überproportionale Änderung des Massesignals erzeugt. Eine kleine Veränderung der Feuchte erzeugt somit eine große Abweichung des Massesignals von der tatsächlichen Masse. Ein weiterer wesentlicher Nachteil ist, dass die Tabletten bei der kapazitiven Messung mit hoher Geschwindigkeit durch einen Messspalt oder Messrohr geführt werden müssen. Das kann bei kleinsten Störungen zur Verstopfung und Unterbrechung des Transportweges führen.

Die US 6,163,158 beschreibt ein Konzept zum Erfassen mindestens einer Eigenschaft eines Stoffes durch Auswertung der durch die Anwesenheit des Stoffes verursachten Verstimmung eines HF-Resonators, dem Mikrowellen zugeführt werden und von dem ein hochfrequentes, von dem Stoff beeinflusstes Signal abgenommen wird, dessen Resonanzfrequenz- Verschiebung und Dämpfung gegenüber einem von dem Stoff unbeeinflussten Signal erfasst wird, wobei dem Resonator Mikrowellen mit mindestens zwei unterschiedlichen Frequenzen zugeführt werden. Die Resonanzfrequenz-Verschiebungen werden durch Vergleich der von dem Stoff unbeeinflussten und beeinflussten Resonanzkurven des Resonators erfasst. Die Dämpfung wird durch Vergleich der Amplituden der Resonanzkurven bei den Frequenzen der zugeführten Mikrowellen erfasst.

Es kann als eine Aufgabe von Ausführungsbeispielen der vorliegenden Erfindung betrachtet werden, den vorbekannten Stand der Technik betreffend die Bestimmung einer für ein Objekt charakteristischen Größe, wie z.B. dessen Masse, weiterzubilden.

Dem wird durch Vorrichtungen und Verfahren mit den Merkmalen der unabhängigen Patentansprüche Rechnung getragen. Vorteilhafte Ausgestaltungen und Weiterbildungen sind Gegenstand der abhängigen Ansprüche.

Gemäß einem ersten Aspekt schaffen Ausführungsbeispiele eine Vorrichtung zur Ermittlung einer für ein Objekt charakteristischen Größe. Die Vorrichtung umfasst dazu eine Signalerzeugungseinheit, die ausgebildet ist, um ein Eingangssignal zu erzeugen. Ferner umfasst die Vorrichtung ein Hochfrequenzfilter mit einem Eingang für das Eingangssignal, einem Ausgang für ein Ausgangssignal und mit einem Bereich, z.B. einer Oberfläche, für eine Wechselwirkung zwischen einem elektromagnetischen Streufeld des Hochfrequenzfilters und dem Objekt. Außerdem ist eine Signalauswerteeinheit vorgesehen, die ausgebildet ist, um aus dem Eingangssignal und dem Ausgangssignal des Hochfrequenzfilters eine Übertragungscharakteristik des Hochfrequenzfilters als Maß für die charakteristische Größe des Objekts zu ermitteln.

Gemäß Ausführungsbeispielen kann die Übertragungscharakteristik eine Übertragungsfunktion des Hochfrequenzfilters und/oder eine Signallaufzeit durch das Hochfrequenzfilter umfassen.

Ausführungsbeispiele basieren somit auf dem Gedanken, die charakteristische Größe des Objekts aus einer für das Objekt charakteristischen Beeinflussung der Übertragungscharakteristik des Hochfrequenzfilters zu ermitteln. Während der Wechselwirkung zwischen Objekt und elektromagnetischem Streufeld befindet sich das Objekt gemäß manchen Ausführungsbeispielen an bzw. unmittelbar auf einer Oberfläche des Hochfrequenzfilters. Letzteres kann beispielsweise als Hohlraum- oder Koaxialresonator ausgebildet sein. Die Oberfläche des Filters für die Wechselwirkung mit dem Messobjekt kann beispielsweise eine für elektromagnetische Strahlung möglichst transparente Seite eines Filtergehäuses, wie z.B. eine Keramikfläche oder ein anderes geeignetes Material umfassen.

Gemäß manchen Ausführungsbeispielen kann zusätzlich eine Transporteinrichtung vorgesehen sein, die ausgebildet ist, um das Objekt an der Oberfläche des Hochfrequenzfilters entlangzuführen. Beispiele für Transporteinrichtungen sind Rohre oder Rinnen, in denen das Objekt mit vergleichsweise hoher Geschwindigkeit an der Oberfläche des Hochfrequenzfilters vorbeigeführt werden kann, um kurzzeitig mit dessen elektromagnetischem Streufeld in Wechselwirkung zu treten. Dabei kann die Filteroberfläche einen Teil der Transporteinrichtung, z.B. eine Rohr- oder Rinnenwand, bilden. Somit können hohe Massestromtransportgeschwindigkeiten, also hohe Produktionsgeschwindigkeiten erzielt werden. Andere Beispiele für Transporteinrichtungen umfassen auch Förderbänder oder dergleichen.

Bevorzugt ist die Transporteinrichtung ausgebildet, um das Objekt die Oberfläche unmittelbar berührend entlang der Oberfläche des Hochfrequenzfilters zu bewegen. Das Objekt steht während der Messung also in berührendem Kontakt mit der Oberfläche des Filters, indem es beispielsweise darauf abrollt oder darüber geschoben wird. Durch die Berührung können besonders exakte Messergebnisse für die charakteristische Größe erzielt werden.

Bei manchen Ausführungsbeispielen kann die charakteristische Größe eine Masse und/oder Feuchte des Objekts umfassen. Von der durch das Objekt veränderten Übertragungscharakteristik des Hochfrequenzfilters kann also auf die Masse und/oder Feuchte des vorbeigeführten Objekts geschlossen werden. Andere charakteristische Größen, wie z.B. Größe, Form, etc., sind selbstverständlich ebenfalls denkbar.

Das Objekt selbst kann gemäß manchen Ausführungsbeispielen wenigstens eine portionierte Feststoffeinheit, wie z.B. eine Tablette, eine Kapsel, etc., umfassen. Somit können Ausführungsbeispiele die Effizienz von Medikamentenproduktion erhöhen.

Bei einigen Ausführungsformen kann die Signalauswerteeinheit ausgebildet sein, um die Übertragungscharakteristik durch eine Ermittlung einer Laufzeit- bzw. Phasendifferenz zwischen dem Eingangssignal und dem Ausgangssignal zu bestimmen. Die Phasendifferenz kann dann als Maß für charakteristische Größe des Objekts herangezogen werden. Dies kann vorteilhaft zu hohen Empfindlichkeiten und damit hohe Messgenauigkeiten führen.

Gemäß manchen Ausführungsbeispielen ist die Signalerzeugungseinheit ausgebildet, um das Eingangssignal mit einer (Träger-) Frequenz in einem Frequenzbereich von 300 MHz bis 300 GHz bereitzustellen. Dabei kann es sich je nach Ausführungsform um ein monofrequentes oder um ein breitbandiges Eingangssignal handeln.

Im letzteren Fall kann die Signalerzeugungseinheit ausgebildet sein, um das Eingangssignal für das Hochfrequenzfilter durch Mischen eines Basisbandeingangssignals mit einem hochfrequenten Trägersignal im Durchlassbereich des Hochfrequenzfilters zu erzeugen. Das Hochfrequenzfilter kann also als Bandpass ausgebildet sein. Insbesondere kann die Signalerzeugungseinheit ausgebildet sein, um das Trägersignal mit einer Trägerfrequenz in einem Frequenzbereich von 300 MHz bis 300 GHz bereitzustellen. Dabei kann die Trägerfrequenz beispielsweise abhängig von einer Signalbandbreite des Basisbandeingangssignals gewählt werden - je größer dessen Bandbreite, desto höher die Trägerfrequenz.

Bei manchen Ausführungsbeispielen ist die Signalerzeugungseinheit ausgebildet, um das Basisbandeingangssignal mit einer Signalbandbreite von wenigstens 10 MHz zu erzeugen. Es handelt sich hier also nicht nur um ein Trägersignal mit einer diskreten Trägerfrequenz, sondern um ein vergleichsweise breitbandiges auf die Trägerfrequenz auf moduliertes Signal. Durch den daraus resultierenden und eine Mehrzahl von Frequenzen umfassenden Spektralbereich der Übertragungscharakteristik kann eine hohe Messgenauigkeit erreicht werden. Es können Amplituden- und/oder Phasengang für die ganze Signalbandbreite oder einen Teil davon, umfassend eine Mehrzahl von Frequenzen, als Objektcharakteristikum ausgewertet werden.

Beispielsweise kann bei Ausführungsbeispielen eine sogenannte Multicarrier-Modulation, insbesondere ein orthogonales Frequenzmultiplexverfahren (OFDM = Orthogonal Frequency-Division Multiplexing), zum Einsatz kommen. Bereits bei dem breitbandigen Basisbandeingangssignal kann es sich also um ein OFDM Signal im Basisband handeln. Selbstverständlich kommen auch andere geeignete Basisbandsignale in Frage, wie z.B. Pseudo-Zufallssequenzen.

Zur Bandbegrenzung des Eingangssignals kann die Signalerzeugungseinheit ausgebildet sein, um das Basisbandeingangssignal mittels einer Fensterfunktion, insbesondere einer Tuckey-Fensterfunktion, zu gewichten. Selbstverständlich können auch andere geeignete Fensterfunktionen zur Bandbegrenzung eingesetzt werden.

Betreffend den Ausgang des Hochfrequenzfilters kann die Signalauswerteeinheit ausgebildet sein, um durch Mischen des Ausgangssignals mit dem Trägersignal ein Basisbandausgangssignal zu erzeugen und, um aus dem Basisbandeingangssignal und dem Basisbandausgangssignal die Übertragungscharakteristik zu ermitteln. Durch die Ermittlung der Übertragungscharakteristik im Basisbandbereich kann auf aufwändige und kostspielige HF-Schaltungsanordnungen verzichtet werden.

Insbesondere kann die Signalauswerteeinheit ausgebildet sein, um die Übertragungscharakteristik durch Division des Basisbandausgangs- und des Basisbandeingangssignals oder deren jeweiligen Frequenzspektren zu ermitteln. Alternativ oder zusätzlich kann die Signalauswerteeinheit ausgebildet sein, um die Übertragungscharakteristik durch einen Phasenvergleich zwischen Basisbandausgangs- und des Basisbandeingangssignal zu ermitteln. Dies kann beispielsweise mittels eines Digitalen Signal Prozessors (DSP) oder eines Field Programmable Gate Arrays (FPGA) implementiert werden.

Gemäß manchen Ausführungsbeispielen kann die Signalauswerteeinheit ausgebildet sein, um die Übertragungscharakteristik mit einer für das Objekt vorbestimmten Referenzübertragungscharakteristik zu vergleichen und eine Fehlermeldung auszugeben, wenn ein Unterschied zwischen der Übertragungscharakteristik und der Referenzübertragungscharakteristik oberhalb eines Schwellenwerts liegt. Damit ist eine effiziente Aussortierung von fehlerhaften Objekt möglich.

Gemäß einem weiteren Aspekt schaffen Ausführungsbeispiele eine Vorrichtung zur Ermittlung einer für wenigstens eine Tablette charakteristischen Größe. Die Vorrichtung umfasst eine Basisbandsignalerzeugungseinheit, die ausgebildet ist, um ein Basisbandeingangssignal mit einer Signalbandbreite von wenigstens 10 MHz zu erzeugen, eine Hochfrequenzträgersignalerzeugungseinheit, die ausgebildet ist, um ein Trägersignal in einem Frequenzbereich von 300 MHz bis 300 GHz zu erzeugen, eine Mischereinheit, die ausgebildet ist, um aus dem Basisbandeingangssignal und dem Trägersignal Hochfrequenzeingangssignal zu erzeugen und ein Hochfrequenzfilter mit einem Eingang für das Hochfrequenzeingangssignal, einem Ausgang für ein Hochfrequenzausgangssignal und mit einer Filteroberfläche. Ferner ist eine Transporteinrichtung vorgesehen, die ausgebildet ist, um die wenigstens eine Tablette an der Oberfläche des Hochfrequenzfilters für eine Wechselwirkung zwischen einem elektromagnetischen Streufeld des Hochfrequenzfilters und der wenigstens eine Tablette vorbeizuführen. Die Mischereinheit ist ausgebildet, um aus dem Hochfrequenzausgangssignal und dem Trägersignal ein Basisbandausgangssignal zu erzeugen. Die Vorrichtung umfasst auch eine Signalauswerteeinheit, die ausgebildet ist, um aus dem Basisbandeingangssignal und dem Basisbandausgangssignal eine eine Mehrzahl von Frequenzen umfassende Übertragungscharakteristik des Hochfrequenzfilters als Maß für die charakteristische Größe der wenigstens einen Tablette ermitteln.

Gemäß einem noch weiteren Aspekt schaffen Ausführungsbeispiele Verfahren zur Ermittlung einer für ein Objekt charakteristischen Größe. Das Verfahren umfasst ein Erzeugen eines Eingangssignals für ein Hochfrequenzfilter; ein Herstellen einer Wechselwirkung zwischen dem Objekt und einem durch das Eingangssignal hervorgerufenen elektromagnetischen Streufeld des Hochfrequenzfilters, und ein Ermitteln, aus dem Eingangssignal und einem Ausgangssignal des Hochfrequenzfilters, einer Übertragungscharakteristik des Hochfrequenzfilters als Maß für die charakteristische Größe.

Gemäß manchen Ausführungsbeispielen umfasst das Herstellen der Wechselwirkung ein Bewegen des Objekts entlang der Oberfläche des Hochfrequenzfilters, insbesondere ein berührendes Bewegen.

Gemäß manchen Ausführungsbeispielen kann die Übertragungscharakteristik durch eine Bestimmung einer Laufzeit- bzw. Phasendifferenz zwischen dem Eingangssignal und dem Ausgangssignal ermittelt werden. Ergänzend oder alternativ kann die Übertragungscharakteristik durch Bestimmen einer Übertragungsfunktion für einen Frequenzbereich umfassend eine Mehrzahl von Frequenzen ermittelt werden.

Als charakteristische Größe kann gemäß manchen Ausführungsbeispielen eine Masse des Objekts bestimmt werden basierend auf der während der Wechselwirkung zwischen dem Objekt und dem elektromagnetischen Streufeld ermittelten Übertragungscharakteristik und basierend auf einer Referenzübertragungscharakteristik des Hochfrequenzfilters ohne Wechselwirkung zwischen dem Objekt und dem elektromagnetischen Streufeld des Hochfrequenzfilters.

Bei anderen Ausführungsformen kann die während der Wechselwirkung zwischen dem Objekt und dem elektromagnetischen Streufeld ermittelte Übertragungscharakteristik mit einem Vergleich mit einer für das Objekt bzw. die Art des Objekts geltenden Soll-Übertragungscharakteristik (oder Referenzübertragungscharakteristik) verglichen werden. Liegt eine Abweichung der gemessenen Übertragungscharakteristik von der Soll-Übertragungscharakteristik innerhalb eines zulässigen Toleranzbereichs, wird das Objekt, z.B. ein oder mehrere Tabletten, zur Weiterverarbeitung freigegeben. Anderenfalls erfolgt eine Fehlermeldung.

Vorteile von Ausführungsbeispielen können darin gesehen werden, dass diese leicht in bestehende Transportvorrichtungen von Tablettenpressen eingefügt werden können. Es braucht keine technisch aufwendige und den Transportstrom verzögernde Einzelpositionierung von Prüfkörpern vorgenommen werden. Weiter erlaubt der vorgeschlagene Ansatz mit einer schnellen digitalen Signalverarbeitung sehr hohe Messraten zu realisieren.

Einige beispielhafte Ausführungsbeispiele der vorliegenden Erfindung werden nachfolgend bezugnehmend auf die beiliegenden Figuren näher erläutert. Es zeigen:

Fig. 1        ein Blockdiagram einer Vorrichtung zur Ermittlung einer Masse und/oder Feuchte wenigstens einer Tablette;

Fig. 2a      Tuckey-Fensterfunktion zur Bandbegrenzung;

Fig. 2b      einen Verlauf einer durch Kalibrierung erhaltenen Referenzübertragungscharakteristik;

Fig. 3      ein schematisches Flussdiagramm eines möglichen Verfahrens zur Ermittlung einer Masse und/oder Feuchte wenigstens einer Tablette;

Fig. 4a,b      Blockdiagramme von Vorrichtungen zur Ermittlung einer Masse und/oder Feuchte wenigstens einer Tablette;

Fig. 5      Verlauf einer gemessenen Phasendifferenz aufgetragen über Frequenz; und

Fig. 6      ein schematisches Flussdiagramm eines weiteren möglichen Verfahrens zur Ermittlung einer Masse und/oder Feuchte wenigstens einer Tablette.

Verschiedene Ausführungsbeispiele werden nun ausführlicher unter Bezugnahme auf die beiliegenden Zeichnungen beschrieben, in denen einige Ausführungsbeispiele dargestellt sind. In den Figuren können die Dickenabmessungen von Linien, Schichten und/oder Regionen um der Deutlichkeit Willen übertrieben dargestellt sein.

Bei der nachfolgenden Beschreibung der beigefügten Figuren, die lediglich einige exemplarische Ausführungsbeispiele zeigen, können gleiche Bezugszeichen gleiche oder vergleichbare Komponenten bezeichnen. Ferner können zusammenfassende Bezugszeichen für Komponenten und Objekte verwendet werden, die mehrfach in einem Ausführungsbeispiel oder in einer Zeichnung auftreten, jedoch hinsichtlich eines oder mehrerer Merkmale gemeinsam beschrieben werden. Komponenten oder Objekte, die mit gleichen oder zusammenfassenden Bezugszeichen beschrieben werden, können hinsichtlich einzelner, mehrerer oder aller Merkmale, beispielsweise ihrer Dimensionierungen, gleich, jedoch gegebenenfalls auch unterschiedlich ausgeführt sein, sofern sich aus der Beschreibung nicht etwas anderes explizit oder implizit ergibt.

Obwohl Ausführungsbeispiele auf verschiedene Weise modifiziert und abgeändert werden können, sind Ausführungsbeispiele in den Figuren als Beispiele dargestellt und werden hierin ausführlich beschrieben. Es sei jedoch klargestellt, dass nicht beabsichtigt ist, Ausführungsbeispiele auf die jeweils offenbarten Formen zu beschränken, sondern dass Ausführungsbeispiele vielmehr sämtliche funktionale und/oder strukturelle Modifikationen, Äquivalente und Alternativen, die im Bereich der Erfindung liegen, abdecken sollen. Gleiche Bezugszeichen bezeichnen in der gesamten Figurenbeschreibung gleiche oder ähnliche Elemente.

Man beachte, dass ein Element, das als mit einem anderen Element "verbunden" oder "verkoppelt" bezeichnet wird, mit dem anderen Element direkt verbunden oder verkoppelt sein kann oder dass dazwischenliegende Elemente vorhanden sein können. Wenn ein Element dagegen als "direkt verbunden" oder "direkt verkoppelt" mit einem anderen Element bezeichnet wird, sind keine dazwischenliegenden Elemente vorhanden. Andere Begriffe, die verwendet werden, um die Beziehung zwischen Elementen zu beschreiben, sollten auf ähnliche Weise interpretiert werden (z.B., "zwischen" gegenüber "direkt dazwischen", "angrenzend" gegenüber "direkt angrenzend" usw.).

Die Terminologie, die hierin verwendet wird, dient nur der Beschreibung bestimmter Ausführungsbeispiele und soll die Ausführungsbeispiele nicht beschränken. Wie hierin verwendet, sollen die Singularformen "einer," "eine", "eines" und "der, die, das" auch die Pluralformen beinhalten, solange der Kontext nicht eindeutig etwas anderes angibt. Ferner sei klargestellt, dass die Ausdrücke wie z.B. "beinhaltet", "beinhaltend", aufweist" und/oder "aufweisend", wie hierin verwendet, das Vorhandensein von genannten Merkmalen, ganzen Zahlen, Schritten, Arbeitsabläufen, Elementen und/oder Komponenten angeben, aber das Vorhandensein oder die Hinzufügung von einem bzw. einer oder mehreren Merkmalen, ganzen Zahlen, Schritten, Arbeitsabläufen, Elementen, Komponenten und/oder Gruppen davon nicht ausschließen.

Solange nichts anderes definiert ist, haben sämtliche hierin verwendeten Begriffe (einschließlich von technischen und wissenschaftlichen Begriffen) die gleiche Bedeutung, die ihnen ein Durchschnittsfachmann auf dem Gebiet, zu dem die Ausführungsbeispiele gehören, beimisst. Ferner sei klargestellt, dass Ausdrücke, z.B. diejenigen, die in allgemein verwendeten Wörterbüchern definiert sind, so zu interpretieren sind, als hätten sie die Bedeutung, die mit ihrer Bedeutung im Kontext der einschlägigen Technik konsistent ist, und nicht in einem idealisierten oder übermäßig formalen Sinn zu interpretieren sind, solange dies hierin nicht ausdrücklich definiert ist.

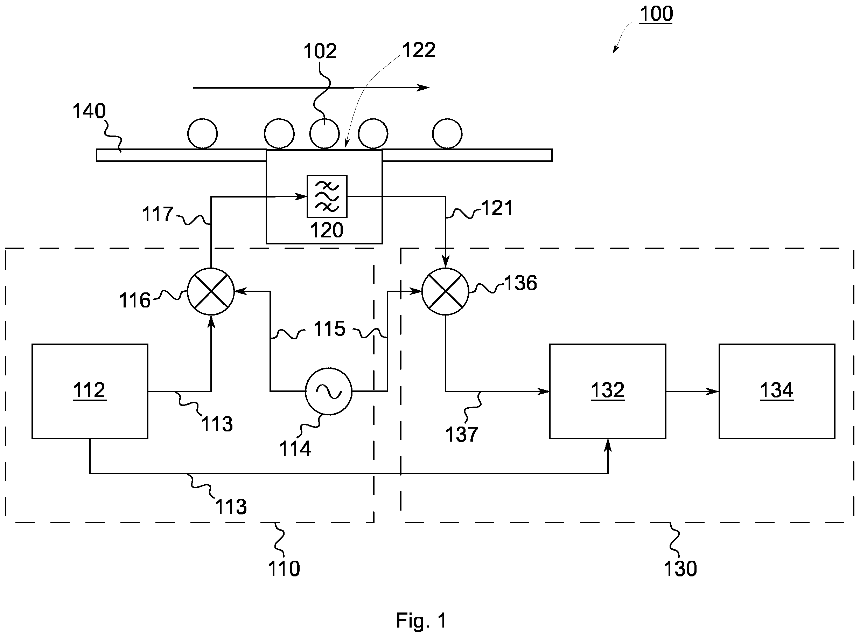

Die **Fig. 1** zeigt ein Blockdiagram einer Vorrichtung 100 zur Ermittlung einer Masse und/oder Feuchte von portionierten Wirkstoffeinheiten 102, wie z.B. Tabletten, Kapseln, Dragees, etc.

Die Vorrichtung 100 umfasst eine Signalerzeugungseinheit 110, ein mit letzterer gekoppeltes Hochfrequenzfilter (HF-Filter) 120 und eine wiederum damit gekoppelte Signalauswerteeinheit 130. Das HF-Filter 120 hat einen Eingang für ein von der Signalerzeugungseinheit 110 erzeugtes HF-Eingangssignal 117, einen Ausgang für ein aus dem HF-Eingangssignal 117 gefiltertes HF-Ausgangssignal 121 und eine Oberfläche 122 für eine Wechselwirkung zwischen einem elektromagnetischen Streufeld des HF-Filters 120 und den portionierten Wirkstoffeinheiten 102. Der Ausgang des HF-Filters 120 ist mit einem Eingang der Signalauswerteeinheit 130 verbindbar bzw. verbunden.

In dem gezeigten Ausführungsbeispiel werden die portionierten Wirkstoffeinheiten 102 mittels einer Transporteinrichtung 140 mit möglichst hoher Geschwindigkeit berührend entlang der Filteroberfläche 122 geführt. In anderen Worten stehen Wirkstoffeinheiten 102 und Filteroberfläche 122 bei der Messung also in Berührung miteinander. Die Transporteinrichtung 140 kann beispielsweise ein Förderband, eine Transportrinne, ein Transportrohr oder dergleichen umfassen, um die Wirkstoffeinheiten 102 in unmittelbaren Kontakt mit der Filteroberfläche bzw. Filtergehäuseoberfläche 122 zu bringen.

Das HF-Filter 120, welches als Bandpassfilter ausgebildet ist, kann beispielsweise als Hohlraum- oder Koaxialresonator implementiert sein. Es kann für eine Mittenfrequenz des HF-Eingangssignals 117 in z.B. einem Bereich von 300 MHz bis 300 GHz ausgelegt sein. Sein Durchlassbereich um die Mittenfrequenz ist entsprechend einer Bandbreite des HF-Eingangssignals 117 beispielsweise wenigstens 10 MHz, wenigstens 50 MHz, oder wenigstens 100 MHz. Die Filteroberfläche 122 kann beispielsweise als Keramikoberfläche ausgelegt sein und Teil einer Transportröhre oder -rinne sein, mittels der die Wirkstoffeinheiten 102 an dem HF-Filter 120 für die Messung berührend vorbeigeführt werden. Das HF-Filter 120 kann so konstruiert sein, dass ein Teil des elektrischen Feldes das zu messende Material durchdringen kann. Dafür kann ein umgebendes Filtergehäuse einseitig offen sein oder eine Durchgangsöffnung aufweisen.

Die Signalerzeugungseinheit 110 ist gemäß dem hier gezeigten Ausführungsbeispiel ausgebildet, um das HF-Eingangssignal 117 für das HF-Filter 120 aus einem Basisbandeingangssignal 113 mit Tiefpassverhalten zu erzeugen. Dazu umfasst die Signalerzeugungseinheit 110 eine Basisbandsignalerzeugungseinheit 112, die ausgebildet ist, um das Basisbandeingangssignal 113 mit einer Signalbandbreite von wenigstens 10 MHz, wenigstens 50 MHz, oder wenigstens 100 MHz zu erzeugen. An dieser Stelle sei angemerkt, dass die Signalbandbreite abhängig von der Geschwindigkeit der Wirkstoffeinheiten 102 gewählt werden kann. Je schneller sich die Wirkstoffeinheiten 102 bewegen, desto größer sollte die Bandbreite gewählt werden. Umgekehrt kann bei langsamer Bewegung die Signalbandbreite kleiner ausfallen. Die Basisbandsignalerzeugungseinheit 112 kann in manchen Ausführungsformen Digital-Analog Wandler (D/A-Wandler) aufweisen, um aus einem digitalen Basisbandsignal ein analoges Basisbandsignal zu erzeugen.

Die Basisbandsignalerzeugungseinheit 112 kann zur Erzeugung des Basisbandeingangssignals 113 beispielsweise eine oder mehrere programmierbare Hardwarekomponenten wie DSPs, ASICs, oder FPGAs umfassen. Bei dem Basisbandeingangssignal 113 kann es sich gemäß manchen Ausführungsbeispielen um ein mit einer Fensterfunktion, z.B. einer Tuckey-Fensterfunktion, gewichtetes Basisbandsignal handeln.

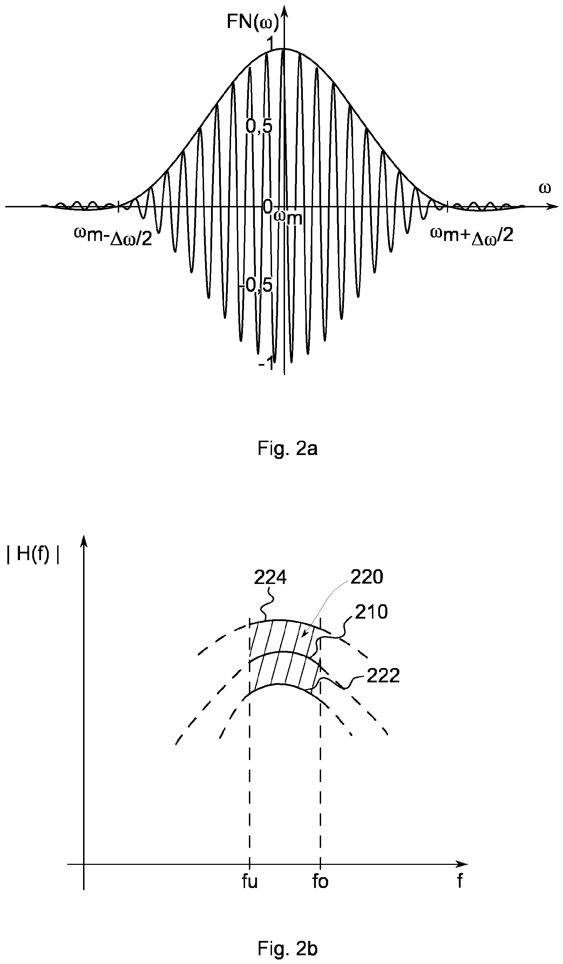

Dazu zeigt **Fig. 2a** beispielhaft den Frequenzverlauf der Tuckey-Fensterfunktion aufgetragen über der Kreisfrequenz $\omega = 2\pi f$. Die Größen $\omega_m$ und $\Delta\omega$ bezeichnen dabei die Mittenfrequenz bzw. die Bandbreite des Signals. Die Tuckey-Gewichtung zeichnet sich durch eine gute Kombination zwischen Frequenz- und Zeitverhalten aus. Typische Bandbreiten können einige hundert MHz sein. Die Mittenfrequenz $\omega_m$ kann durch eine Frequenzumsetzung fast beliebig gewählt werden, vorzugsweise jedoch in einem Bereich 300 MHz bis 300 GHz.

Für diesen speziellen Fall kann Spektrum und Zeitverlauf des Messsignals 113 bzw. 117 analytisch angegeben werden.

$$F_N(\omega) = \begin{cases} \dfrac{1}{2} * \left(1 + \cos\dfrac{2\pi(\omega - \omega_m)}{\Delta\omega}\right) & f\ddot{u}r \ |\omega - \omega_m| \leq \dfrac{\Delta\omega}{2} \\ 0 & sonst \end{cases}$$

Die zugeordnete Zeitbereichsfunktion $F_N(t)$ lässt sich leicht durch eine inverse Fourier-Transformation berechnen:

$$f_N(t) = \frac{\pi^2 * sin\dfrac{\Delta\omega * t}{2}}{\dfrac{\Delta\omega * t}{2} * \left(\pi^2 - \dfrac{\Delta\omega^2 * t^2}{4}\right)} * e^{j\omega_m t}$$

Alternative Breitbandsignale wie FM-Chirps oder Mehrträgersignale (z.B. OFDM-Signale) können natürlich ebenfalls verwendet werden.

Um aus dem Basisbandeingangssignal 113 das HF-Eingangssignal 117 mit der Mitten- bzw. Trägerfrequenz $\omega_m$ zu erhalten, weist die Signalerzeugungseinheit 110 außerdem eine Hochfrequenzträgersignalerzeugungseinheit 114 auf, die ausgebildet ist, um ein HF-Trägersignal 115 z.B. in einem Frequenzbereich von 300 MHz bis 300 GHz zu erzeugen. Dazu kann die Hochfrequenzträgersignalerzeugungseinheit 114 eine Oszillatorschaltung umfassen. Ferner ist in der Signalerzeugungseinheit 110 zur Frequenzumsetzung eine Aufwärtsmischereinheit 116 vorgesehen, die ausgebildet ist, um aus dem breitbandigen und bandbegrenzten Basisbandeingangssignal 113 und dem HF-Trägersignal 115 das breitbandige und bandbegrenzte HF-Eingangssignal 117 für das HF-Filter 120 zu erzeugen. Das so erhaltene bandbegrenzte HF-Signal 117 kann dann nach entsprechender Konditionierung (Verstärkung, Anpassung, ggf. Filterung, etc.) auf die Testanordnung mit dem HF-Filter 120 gegeben werden.

Durch Speisen des HF-Filters 120 mit dem HF-Eingangssignal 117 entsteht in dem HF-Filter 120 und in dessen Umgebung ein elektromagnetisches Feld. Die entlang der Filteroberfläche vorbeigeführten Wirkstoffeinheiten 102 beeinflussen dieses elektromagnetische Feld für die Wirkstoffeinheiten charakteristisch und verändern damit eine Übertragungscharakteristik des HF-Filters 120, wie z.B. dessen Amplitudengang (Betragsfrequenzgang) und/oder Phasengang (Phasenfrequenzgang).

Die veränderte Übertragungscharakteristik des HF-Filters 120 als Maß z.B. für die Masse und/oder Feuchte der Wirkstoffeinheiten 102 kann von der Signalauswerteinheit 130 ermittelt werden. Auch der Empfang des durch die Testanordnung gefilterten Signals 121 kann im Zeitbereich erfolgen. Dazu ist in dem dargestellten Ausführungsbeispiel eine optionale Abwärtsmischereinheit 136 vorgesehen, um aus dem HF-Ausgangssignal 121 und dem HF-Trägersignal 115 der Hochfrequenzträgersignalerzeugungseinheit 114 ein Zwischenfrequenz- oder Basisbandausgangssignal 137 mit handhabbaren Frequenzen zu erzeugen. Mit entsprechend schnellen Digital Wandlern (A/D-Wandlern) 132 könnte auf die Abwärtsmischereinheit 136 auch verzichtet werden.

Die Signalauswerteinheit 130 ist in dem dargestellten Beispiel ausgebildet, um aus dem Basisbandeingangssignal (Referenzsignal) 113 und dem Basisbandausgangssignal (Empfangssignal) 137 eine Übertragungscharakteristik des HF-Filters 120 als Maß für die charakteristische Größe (Masse/Feuchte) der Wirkstoffeinheiten 102 zu ermitteln. Dazu können in manchen Ausführungsbeispielen einem Analog-Digital Wandler (A/D-Wandler) 132 die beiden (analogen) Basisbandsignale 113 und 137 zugeführt werden, bevor eine digitale Signalverarbeitungseinheit 134, z.B. in Form eines FPGA, eine digitale Signalverarbeitung der resultierenden digitalen Basisbandsignale vornimmt. In anderen Ausführungsformen könnte die Signalauswerteinheit 130 aber auch einen Speicher für das bekannte Basisbandeingangssignal (Referenzsignal) 113 umfassen. Die gewünschte Information erhält man z.B. durch Division von Empfangsspektrum durch Referenzspektrum innerhalb der Messbandbreite.

Mit modernen A/D-Wandlern sind Abtastraten bis über 1 GSample/sec. möglich. Entsprechend kann die Messbandbreite des Signals 113 bzw. 117 mehrere 100 MHz betragen. Die Messdauer kann mit modernen FPGAs leicht auf unter $1\mu s$ reduziert werden.

Während der Bewegung der Wirkstoffeinheiten 102 entlang der Oberfläche des HF-Filters 120 kann von der digitalen Signalverarbeitungseinheit 134 mittels der digitalisierten Signale 113 und 137 in kurzen zeitlichen Abständen die Übertragungsfunktion H(f) des Filters 120 über einen Frequenzbereich mit einer Mehrzahl von Frequenzen bestimmt werden. Diese diskreten Werte müssen nicht den genauen Zeitpunkt erfasst haben, an dem sich eine Wirkstoffeinheit 102 am Ort der höchsten Messempfindlichkeit befand. Deshalb kann aus den diskreten Messwerten und einem bekannten Empfindlichkeitsverlauf des HF-Filters 120 die maximale Veränderung der Übertragungsfunktion errechnet werden. Dieser ermittelte Punkt der größten Beeinflussung der Übertragungsfunktion kann als Maß für die Masse der Wirkstoffeinheiten 102 dienen.

Als charakteristische Größe kann gemäß manchen Ausführungsbeispielen direkt oder indirekt eine Masse einer Wirkstoffeinheit 102 bestimmt werden basierend auf der während der Wechselwirkung zwischen der Wirkstoffeinheit 102 und dem elektromagnetischen Streufeld ermittelten Übertragungscharakteristik und basierend auf einer Referenzübertragungscharakteristik des HF-Filters 120. Diese Referenzübertragungscharakteristik kann vorab durch Kalibrierung mit Probekörpern, deren Masse genau bekannt ist, erhalten werden. Zum Beispiel kann dazu eine bestimmte Menge von einem zu messenden Tablettentyp zuerst genau ausgewogen werden. Die Variationen der Gewichte der Testtabletten können dabei größer gewählt werden als der Massebereich in dem die später als "gut" deklarierten Wirkstoffeinheiten 102 liegen.

Fig. 2b zeigt hierzu schematisch den Verlauf einer durch Vorabkalibrierung mit bekannten Masseeinheiten erhaltenen Referenzübertragungscharakteristik 210 in einem Frequenzband von einer unteren Frequenz $f_u$ bis zu einer oberen Frequenz $f_o$. Ferner ist ein Toleranzbereich 220 mit betragsmäßigen Unter- und Obergrenzen 222, 224 eingezeichnet.

Während der Produktion kann nun eine während der Wechselwirkung zwischen den Wirkstoffeinheiten 102 und dem elektromagnetischen Streufeld gemessene Übertragungscharakteristik mit der für die Wirkstoffeinheit 102 geltenden Soll- bzw. Referenzübertragungscharakteristik 210 verglichen werden. Liegt eine Abweichung der gemessenen Übertragungscharakteristik von der Soll-Übertragungscharakteristik 210 innerhalb des zulässigen Toleranzbereichs 220, wird eine Wirkstoffeinheit 102 zur Weiterverarbeitung freigegeben. Anderenfalls erfolgt eine Fehlermeldung.

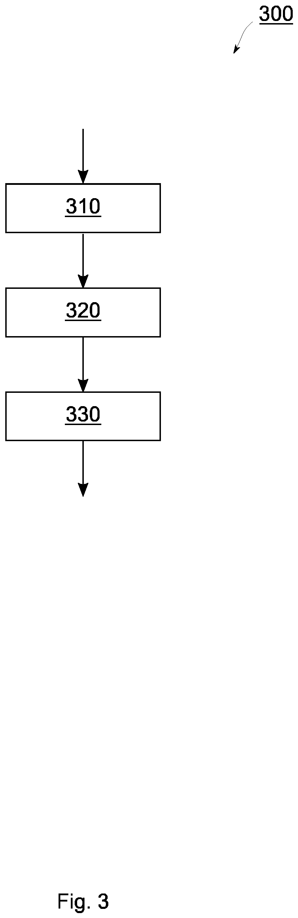

Die **Fig.3** zeigt ein schematisches Ablaufdiagram eines Verfahrens 300, welches die beschriebene Messanordnung 100 ausführen kann.

Das Verfahren 300 umfasst ein Erzeugen 310 eines Eingangssignals 113, 117 für das HF-Filter 120. Dazu kann zunächst ein Basisbandeingangssignal 113 mit einer Signalbandbreite von wenigstens 10 MHz, wenigstens 50 MHz, oder wenigstens 100 MHz erzeugt werden. Ferner kann ein Trägersignal 115 in einem Frequenzbereich von z.B. 300 MHz bis 300 GHz erzeugt werden. Aus dem Basisbandeingangssignal 113 und dem Trägersignal 115 kann anschließend ein breitbandiges Hochfrequenzeingangssignal 117 für das Filter 120 erzeugt werden.

Das Verfahren 300 umfasst ferner ein Herstellen 320 einer Wechselwirkung zwischen wenigstens einer Wirkstoffeinheit 102 und einem durch das Eingangssignal 117 hervorgerufenen elektromagnetischen Streufeld des HF-Filters 120. Dazu kann die wenigstens eine Wirkstoffeinheit 102 an der Oberfläche 122 des HF-Filters 120 für eine Wechselwirkung zwischen dem elektromagnetischen Streufeld des HF-Filters 120 und der wenigstens einen Wirkstoffeinheit 102 z.B. berührend vorbeigeführt werden.

Das Verfahren 300 weist zudem ein Ermitteln 330 einer Übertragungscharakteristik des HF-Filters 120 als Maß für die charakteristische Größe aus dem Eingangssignal 113, 117 und einem Ausgangssignal 121, 137 des HF-Filters 120 auf. Dazu kann beispielsweise aus dem HF-Ausgangssignal 121 und dem Trägersignal 115 ein Basisbandausgangssignal 137 erzeugt werden. Aus dem Basisbandeingangssignal 113 und dem Basisbandausgangssignal 137 kann eine Übertragungscharakteristik des HF-Filters 120 als Maß für die charakteristische Größe der wenigstens einen Wirkstoffeinheit 102 ermittelt werden.

Basierend auf der während der Wechselwirkung zwischen dem Objekt und dem elektromagnetischen Streufeld ermittelten Übertragungscharakteristik und basierend auf einer Referenzübertragungscharakteristik des Hochfrequenzfilters kann nun die Masse der wenigstens einen Wirkstoffeinheit 102 ermittelt werden. Dazu kann die gemessene Übertragungscharakteristik mit einer für die wenigstens eine Wirkstoffeinheit 102 geltende Referenzübertragungscharakteristik verglichen werden. Diese Referenzübertragungscharakteristik kann beispielsweise vorab einkalibriert werden. Liegt eine Abweichung der gemessenen Übertragungscharakteristik von der Soll-Übertragungscharakteristik innerhalb eines zulässigen Toleranzbereichs, wird die wenigstens eine Wirkstoffeinheit 102, z.B. ein oder mehrere Tabletten, zur Weiterverarbeitung freigegeben. Anderenfalls kann eine Fehlermeldung ausgegeben werden.

Die Messung der Übertragungsfunktion H(f) des HF-Filters 120 kann konventionell mit einem Netzwerkanalysator erfolgen, wobei nacheinander bei verschiedenen diskreten Frequenzpunkten Betrag und Phase der interessierenden Übertragungsfunktion aufgenommen werden. Als Anregung werden monofrequente Signale (cw = continuous wave) benützt. Die Übertragungsfunktion kann die Transmissionseigenschaften oder die Reflexionseigenschaften der Testanordnung beschreiben.

Für sehr schnelle Vorgänge, wie der Medikamentenproduktion, ist es allerdings zu langwierig, die Übertragungsfunktion H(f) nacheinander bei verschiedenen Frequenzen zu messen. Hier bietet sich die systemtheoretische gleichwertige Messung im Zeitbereich an, was sich Ausführungsbeispiele der vorliegenden Erfindung zunutze machen. Systeme werden im Zeitbereich durch ihre Impulsantwort beschrieben. Dazu werden kurze trägerfrequente (d.h. auf den Träger 115 aufmodulierte) Pulse verwendet. Sie belegen eine wohldefinierte von Null verschiedene Bandbreite und erzeugen ein ebenso wohldefiniertes Spektrum.

Zur Ermittlung des Einflusses der Wirkstoffeinheiten 102 werden Sende- und Empfangssignal miteinander verglichen. Dies kann zum Beispiel durch Ermittlung der jeweiligen Spektren der beiden Signale 113 und 137 durch Fourier-Transformation erfolgen. Die Übertragungsfunktion H(f) kann durch Division der beiden Spektren erhalten werden. In der Praxis beschränkt man hierbei die Division auf die Signalbandbreite, um Division durch 0 zu vermeiden.

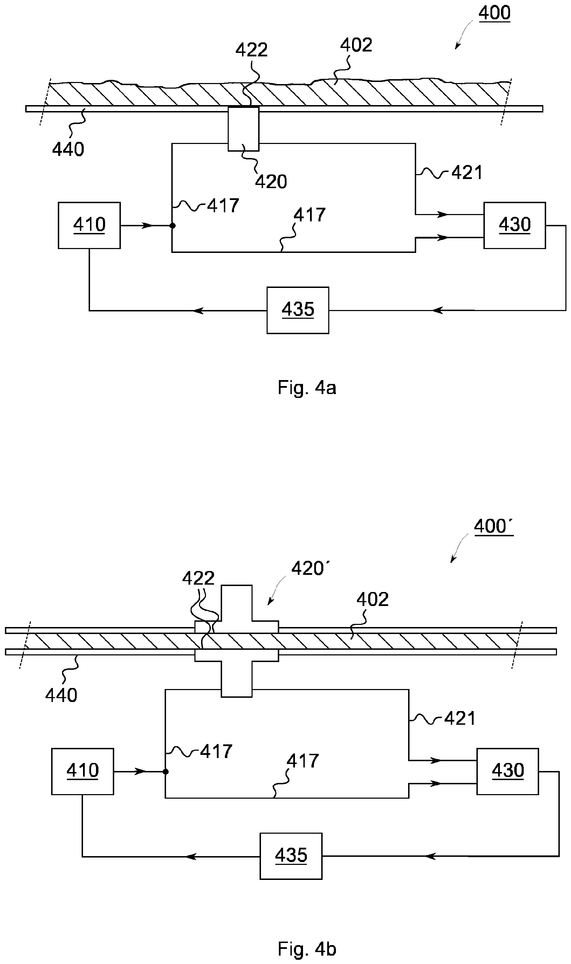

Weitere mögliche Vergleichsbeispiele werden nun anhand der **Fig. 4a, b** beschrieben.

Die Vorrichtung 400 umfasst wieder eine Signalerzeugungseinheit 410, ein mit letzterer gekoppeltes Hochfrequenzfilter (HF-Filter) 420 und eine wiederum damit gekoppelte Signalauswerteeinheit 430. Das HF-Filter 420 hat einen Eingang für ein von der Signalerzeugungseinheit 410 erzeugtes HF-Eingangssignal 417, einen Ausgang für ein gefiltertes HF-Ausgangssignal 421 und eine (geschlossene oder offene) Oberfläche 422 für eine Wechselwirkung zwischen einem elektromagnetischen Streufeld des HF-Filters 420 und den portionierten Wirkstoffeinheiten 402. Der Ausgang des HF-Filters 420 ist mit einem Eingang der Signalauswerteeinheit 430 verbunden.

Die Signalerzeugungseinheit 410 ist ausgebildet, um ein Eingangssignal 417 für das HF-Filter 420 zu erzeugen. Im Gegensatz zur Ausführungsform der Fig. 1 kann es sich hier bei dem Eingangssignal 417 um ein monofrequentes HF-Signal, also beispielsweise das Trägersignal 115, handeln. Die Signalauswerteeinheit 130 ist ausgebildet, um aus dem Eingangssignal 417 und dem Ausgangssignal 421 des HF-Filters 420 eine Übertragungscharakteristik in Form einer Laufzeitdifferenz zwischen den Signalen 417 und 421 als Maß für die charakteristische Größe der portionierten Wirkstoffeinheiten 102 zu ermitteln.

Im Aufbau nach Fig. 4a wird ein Massestrom 402 an dem HF-Filter 420 vorbeigeführt. Die Messung kann dabei wiederum berührend erfolgen. Das HF-Filter 420 kann z.B. in ein einseitig offenes Gehäuse eingebaut sein. Bei Verwendung eines dünnen Netzes 440 zum Massetransport, kann das HF-Filter 420 unter dem Netz montiert werden und die Messung durch das Netz erfolgen. Selbstverständlich kann statt des offenen Gehäuses auch eine Keramikoberfläche oder dergleichen verwendet werden.

Das HF- bzw. Mikrowellensignal 417 wird in der Signalerzeugungseinheit 410 erzeugt und parallel dem HF-Filter 420 und der Signalauswerteeinheit 430 zugeführt. In der Signalauswerteeinheit 430 kann die Phasendifferenz zwischen dem Filterausgangssignal 421 und dem Eingangssignal 417 von der der Signalerzeugungseinheit 410 gebildet werden. Über einen passend eingestellten Regler 435 kann die Frequenz der Signalerzeugungseinheit 410 bzw. des von diesem erzeugten Eingangssignals 417 so nachgeregelt werden, dass die Frequenz des Eingangssignals 417 im Maximum der Übertragungsfunktion der Filterstruktur liegt. Als Kriterium hierfür kann die Stelle maximaler Steigung der gemessenen Kurve für die Phasendifferenz herangezogen werden.

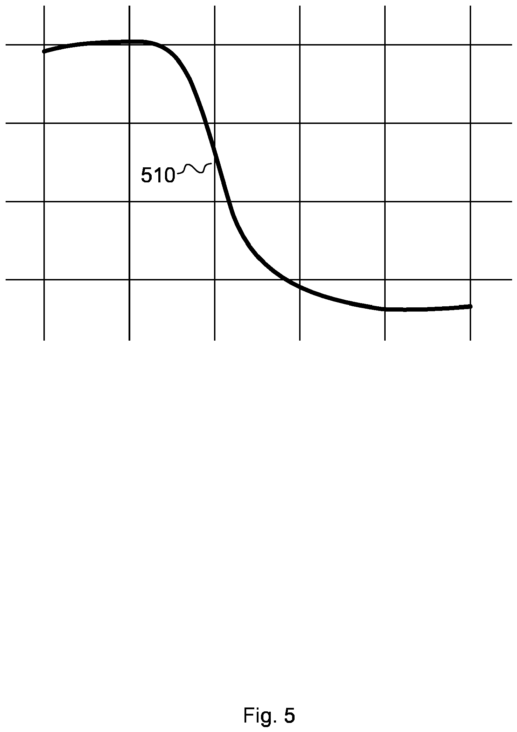

In **Fig. 5** ist ein möglicher Verlauf der gemessen Phasendifferenz des HF-Filters 420 zu sehen. Auf der horizontalen Achse ist dabei die Frequenz und auf der vertikalen Achse die Phasendifferenz aufgetragen. Die Kurve zeigt einen möglichen Verlauf der Phasendifferenz mit dem Punkt größter Steigung bei 510.

Im alternativen Aufbau nach Fig. 4b wird der Massestrom 402 durch einen Sensorkopf 420' geführt, die Messung erfolgt dabei ebenfalls berührend. Der Sensorkopf 420' umfasst eine passend gewählte Filterstruktur, die in ein Gehäuse mit einer Durchgangsöffnung eingebaut sein kann. Die Messung erfolgt analog zum Aufbau aus Fig. 4a. Der Massetransport erfolgt durch hier exemplarisch ein geeignetes Rohr 440.

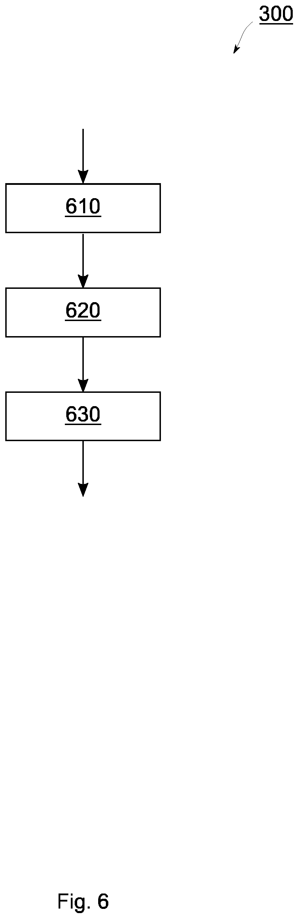

Die **Fig. 6** zeigt ein schematisches Ablaufdiagramm eines Verfahrens 600, welches die Messanordnung 400 ausführen kann.

Das Verfahren 600 umfasst ein Erzeugen 610 eines Eingangssignals 417 für das HF-Filter 420. Dazu kann ein HF-Signal mit einer (diskreten) Frequenz von z.B. 300 MHz bis 300 GHz erzeugt werden.

Das Verfahren 600 umfasst ferner ein Herstellen 620 einer Wechselwirkung zwischen wenigstens einer Wirkstoffeinheit 402 und einem durch das Eingangssignal 417 hervorgerufenen elektromagnetischen Streufeld des HF-Filters 420. Dazu kann die wenigstens eine Wirkstoffeinheit 402 an der Oberfläche 422 des HF-Filters 420 für eine Wechselwirkung zwischen dem elektromagnetischen Streufeld des HF-Filters 420 und der wenigstens einen Wirkstoffeinheit 102 z.B. berührend vorbeigeführt werden.

Das Verfahren 600 weist zudem ein Ermitteln 630 einer Übertragungscharakteristik des HF-Filters 420 als Maß für die charakteristische Größe basierend auf dem Eingangssignal 417 und einem Ausgangssignal 421 des HF-Filters 420. Dazu können die Phasen der HF-Signale 417 und 421, oder die Phasen von daraus abgeleiteten Signalen, verglichen werden. Je größer der Einfluss des Massestroms 402 auf die Signallaufzeit durch das Filter 420, desto größer wird der Phasenunterschied als charakteristisches Maß für den Massestrom 402 ausfallen.

Zusammenfassend betreffen Ausführungsbeispiele der vorliegenden Erfindung ein Konzept zum Messen der Masse und/oder Feuchte von Tabletten oder anderen portionierten Feststoffeinheiten durch berührende Messung mit einer Hochfrequenzfilterstruktur. Dabei können die Tabletten oder portionierten Feststoffeinheiten mit hoher Geschwindigkeit über die Oberfläche eines Hochfrequenzfilters geführt werden. Das Vorhandensein von Materialen im Bereich des Streufeldes bewirkt eine veränderte Phasenverschiebung und/oder veränderte Bedämpfung der Signalkette.

Aus dieser durch den Massenstrom hervorgerufenen Veränderung der Übertragungsfunktion des Hochfrequenzfilters bzw. der Signallaufzeit durch das Filter kann auf die Masse und/oder den Feuchtigkeitsgehalt der Tabletten oder anderen portionierten Feststoffeinheiten geschlossen werden.

Zur Bestimmung der Übertragungsfunktion werden gemäß einem Aspekt auf einen hochfrequenten Träger, dessen Frequenz im Durchlassbereich des Filters liegt, mehrere Signalanteile bestimmter Amplitude und Frequenz, also ein Breitbandsignal, aufmoduliert. Der modulierte Träger wird durch das Filter geleitet und anschließend demoduliert und die resultierenden Signale digitalisiert. Aus den digitalisierten Signalen kann die Übertragungsfunktion des Hochfrequenzfilters durch digitale Signalverarbeitung berechnet werden. Sowohl die Modulation und als auch die digitale Signalverarbeitung können mit so hoher Bandbreite, beispielsweise 200 MHz, erfolgen, dass die mechanische Bewegung der Tabletten bzw. der portionierten Feststoffeinheiten im Vergleich dazu sehr langsam ist. Das heißt, auch bei sehr hohen Transportgeschwindigkeiten durch das Feld des Hochfrequenzfilters wird eine Tablette bzw. portionierte Feststoffeinheit mehrfach vermessen. Mit der Kenntnis der Feldverteilung des Hochfrequenzfilters können für jede Tablettenform bzw. portionierte Feststoffeinheit und jede Transportgeschwindigkeit die genauen relevanten Messwerte errechnet werden. Ein großer Vorteil dieses Messverfahren besteht darin, dass in sehr kurzer Zeit die Übertragungsfunktion der Messanordnung über einen ganzen Frequenzbereich ermittelt werden kann.

Gemäß einem weiteren Aspekt bewirkt das Vorhandensein von Material im Bereich des Streufeldes eine Veränderung der Laufzeit durch die Hochfrequenzfilterstruktur. Einem Detektor wird das Signal durch die Filterstruktur vom Generator und als Referenz das Signal direkt von der Signalerzeugungseinheit zugeführt. Dieses Signal braucht hier nicht breitbandig zu sein. Der Detektor wertet eine Phasendifferenz zwischen den beiden Signalen aus. Über einen passend eingestellten Regler kann die Signalfrequenz der Signalerzeugungseinheit jetzt so nachgeregelt werden, dass die Frequenz im Punkt der maximalen Steigung der gemessenen Kurve für die Phasendifferenz liegt. Die Frequenz des vom Generator gelieferten Signals ist nun ein Maß für die Summe der dielektrischen Eigenschaften des zu messenden Stoffes. Da die Frequenz sehr genau zu messen ist, erreicht man eine hohe Messgenauigkeit. Durch passende Dimensionierung des Reglers ist zusätzlich eine hohe Messgeschwindigkeit erreichbar. Ein wesentlicher Vorteil hier ist die hohe Empfindlichkeit und damit hohe Messgenauigkeit.

Die in der vorstehenden Beschreibung, den nachfolgenden Ansprüchen und den beigefügten Figuren offenbarten Merkmale können sowohl einzeln wie auch in beliebiger Kombination für die Verwirklichung eines Ausführungsbeispiels in ihren verschiedenen Ausgestaltungen von Bedeutung sein und implementiert werden.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Je nach bestimmten Implementierungsanforderungen können Ausführungsbeispiele der vorliegenden Erfindung, zumindest Teile davon, in Hardware oder in Software implementiert sein. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente kann durch einen Prozessor, einen Computerprozessor (CPU = Central Processing Unit), einen Grafikprozessor (GPU = Graphics Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikroprozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardwarekomponente derart zusammenzuwirken, dass eines der hierin beschriebenen Verfahren durchgeführt wird. Ein Ausführungsbeispiel ist somit ein Datenträger (oder ein digitales Speichermedium oder ein computerlesbares Medium), auf dem das Programm zum Durchführen eines der hierin beschriebenen Verfahren aufgezeichnet ist.

Allgemein können Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren durchzuführen, wenn das Programm auf einem Prozessor oder einer programmierbaren Hardwarekomponente abläuft. Der Programmcode oder die Daten kann bzw. können beispielsweise auch auf einem maschinenlesbaren Träger oder Datenträger gespeichert sein. Der Programmcode oder die Daten können unter anderem als Quellcode, Maschinencode oder Bytecode sowie als anderer Zwischencode vorliegen.

Ein Programm gemäß einem Ausführungsbeispiel kann eines der Verfahren während seiner Durchführung beispielsweise dadurch umsetzen, dass dieses Speicherstellen ausliest oder in diese ein Datum oder mehrere Daten hinein schreibt, wodurch gegebenenfalls Schaltvorgänge oder andere Vorgänge in Transistorstrukturen, in Verstärkerstrukturen oder in anderen elektrischen, optischen, magnetischen oder nach einem anderen Funktionsprinzip arbeitenden Bauteile hervorgerufen werden. Entsprechend können durch ein Auslesen einer Speicherstelle Daten, Werte, Sensorwerte oder andere Informationen von einem Programm erfasst, bestimmt oder gemessen werden. Ein Programm kann daher durch ein Auslesen von einer oder mehreren Speicherstellen Größen, Werte, Messgrößen und andere Informationen erfassen, bestimmen oder messen, sowie durch ein Schreiben in eine oder mehrere Speicherstellen eine Aktion bewirken, veranlassen oder durchführen sowie andere Geräte, Maschinen und Komponenten ansteuern.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

**Patentansprüche**

1. Vorrichtung (100) zur Ermittlung einer für ein Objekt (102) charakteristischen Größe, umfassend:

    eine Signalerzeugungseinheit (110), die ausgebildet ist, um ein Eingangssignal (113; 117) zu erzeugen;
    ein Hochfrequenzfilter (120) mit einem Eingang für das Eingangssignal (113; 117;), einem Ausgang für ein Ausgangssignal (121; 137), und mit einem Bereich (122) für eine Wechselwirkung zwischen einem elektromagnetischen Streufeld des Hochfrequenzfilters (120) und dem Objekt (102); und
    eine Signalauswerteeinheit (130), die ausgebildet ist, um aus dem Eingangssignal (113; 117) und dem Ausgangssignal (121; 137) des Hochfrequenzfilters eine Übertragungscharakteristik des Hochfrequenzfilters als Maß für die charakteristische Größe des Objekts (102) zu ermitteln,
    **dadurch gekennzeichnet, dass**

die Signalerzeugungseinheit (110) ausgebildet ist, um das Eingangssignal (117) durch Mischen eines Basisbandeingangssignals (113) mit einem Trägersignal (115) im Durchlassbereich des Hochfrequenzfilters (120) zu erzeugen,

die Signalerzeugungseinheit (110) ausgebildet ist, um das Basisbandeingangssignal (113) mit einer Signalbandbreite von wenigstens 10 MHz zu erzeugen, und

die Signalauswerteeinheit (130) ausgebildet ist, um durch Mischen des Ausgangssignals (121) mit dem Trägersignal (115) ein Basisbandausgangssignal (137) zu erzeugen und, um aus dem Basisbandeingangssignal (113) und dem Basisbandausgangssignal (137) die Übertragungscharakteristik zu ermitteln.

2. Vorrichtung (100) nach Anspruch 1, ferner umfassend:
eine Transporteinrichtung (140), die ausgebildet ist, um das Objekt (102) an einer Oberfläche (122) des Hochfrequenzfilters entlangzuführen.

3. Vorrichtung (100) nach Anspruch 2, wobei die Transporteinrichtung (140) ausgebildet ist, um das Objekt (102) die Oberfläche (122) berührend entlang der Oberfläche zu bewegen.

4. Vorrichtung (100) nach einem der vorhergehenden Ansprüche, wobei die Signalauswerteeinheit (130) ausgebildet ist, um die Übertragungscharakteristik durch eine Ermittlung einer Phasendifferenz zwischen dem Eingangssignal (113; 117) und dem Ausgangssignal (121; 137) zu bestimmen.

5. Vorrichtung (100) nach einem der vorhergehenden Ansprüche, wobei die Signalerzeugungseinheit (110) ausgebildet ist, um das Eingangssignal (117) mit einer Trägerfrequenz in einem Frequenzbereich von 300 MHz bis 300 GHz bereitzustellen.

6. Vorrichtung (100) nach einem der vorhergehenden Ansprüche, wobei die Signalerzeugungseinheit (110) ausgebildet ist, um das Basisbandeingangssignal (113) mittels einer Fensterfunktion, insbesondere einer Tuckey-Fensterfunktion, zu gewichten.

7. Vorrichtung (100) nach einem der vorhergehenden Ansprüche, wobei die Signalauswerteeinheit (130) ausgebildet ist, um die Übertragungscharakteristik für einen Frequenzbereich umfassend eine Mehrzahl von Frequenzen zu bestimmen.

8. Vorrichtung (100) nach einem der vorhergehenden Ansprüche, wobei die Signalauswerteeinheit (130) ausgebildet ist, um die Übertragungscharakteristik mit einer für das Objekt (102) vorbestimmten Referenzübertragungscharakteristik zu vergleichen und eine Fehlermeldung auszugeben, wenn ein Unterschied zwischen der Übertragungscharakteristik und der Referenzübertragungscharakteristik oberhalb eines Schwellenwerts liegt.

9. Vorrichtung (100) nach einem der vorhergehenden Ansprüche, wobei das Objekt (102) wenigstens eine Tablette umfasst und die charakteristische Größe eine Masse und/oder Feuchte der wenigstens einen Tablette ist.

10. Verfahren (300) zur Ermittlung einer für ein Objekt (102) charakteristischen Größe, umfassend:

Erzeugen (310) eines Eingangssignals für ein Hochfrequenzfilter;
Herstellen (320) einer Wechselwirkung zwischen dem Objekt und einem durch das Eingangssignal hervorgerufenen elektromagnetischen Streufeld des Hochfrequenzfilters;
Ermitteln (330), aus dem Eingangssignal und einem Ausgangssignal des Hochfrequenzfilters, einer Übertragungscharakteristik des Hochfrequenzfilters als Maß für die charakteristische Größe,
**dadurch gekennzeichnet, dass**
das Eingangssignal (117; 417) durch Mischen eines Basisbandeingangssignals (113) mit einem Trägersignal (115) im Durchlassbereich des Hochfrequenzfilters (120) erzeugt wird,
das Basisbandeingangssignal (113) mit einer Signalbandbreite von wenigstens 10 MHz erzeugt wird,
das Ausgangssignal (121) mit dem Trägersignal (115) gemischt wird, um ein Basisbandausgangssignal (137) zu erzeugen, und
die Übertragungscharakteristik aus dem Basisbandeingangssignal (113) und dem Basisbandausgangssignal (137) ermittelt wird.

11. Verfahren (300) nach Anspruch 10, wobei das Herstellen (320) der Wechselwirkung eine Bewegung des Objekts entlang der Oberfläche des Hochfrequenzfilters umfasst.

## Claims

1. An apparatus (100) for determining a variable characteristic of an object (102) comprising:

   a signal generation unit (110) configured to generate an input signal (113; 117);
   a high-frequency filter (120) with an input for the input signal (113; 117;), an output for an output signal (121; 137), and with a region (122) for an interaction between an electromagnetic stray field of the high-frequency filter (120) and the object (102); and
   a signal evaluation unit (130) configured to use the input signal (113; 117) and the output signal (121; 137) of the high-frequency filter to determine a transmission characteristic of the high-frequency filter as a measure for the characteristic size of the object (102),
   **characterized in that**
   the signal generation unit (110) is configured to generate the input signal (117) by mixing a baseband input signal (113) with a carrier signal (115) in the pass band of the high-frequency filter (120),
   the signal generation unit (110) is configured to generate the baseband input signal (113) with a signal bandwidth of at least 10 MHz,
   and
   the signal evaluation unit (130) is configured to generate a baseband output signal (137) by mixing the output signal (121) with the carrier signal (115) and to determine the transmission characteristic from the baseband input signal (113) and the baseband output signal (137).

2. The apparatus (100) of claim 1, further comprising:
   a transport device (140) configured to guide the object (102) along a surface (122) of the high-frequency filter.

3. The apparatus (100) of claim 2, wherein the transport device (140) is configured to move the object (102) along the surface in a surface- (122) contacting manner.

4. The apparatus (100) of any of the preceding claims, wherein the signal evaluation unit (130) is configured to establish the transmission characteristic by determining a phase difference between the input signal (113; 117) and the output signal (121; 137).

5. The apparatus (100) of any of the preceding claims, wherein the signal generation unit (110) is configured to provide the input signal (117) with a carrier frequency in a frequency range from 300 MHz to 300 GHz.

6. The apparatus (100) of any of the preceding claims, wherein the signal generation unit (110) is configured to weight the baseband input signal (113) by means of a window function, in particular a Tuckey window function.

7. The apparatus (100) of any of the preceding claims, wherein the signal evaluation unit (130) is configured to establish the transmission characteristic for a frequency range comprising a plurality of frequencies.

8. The apparatus (100) of any of the preceding claims, wherein the signal evaluation unit (130) is configured to compare the transmission characteristic with a reference transmission characteristic pre-established for the object (102) and to output an error message if a difference between the transmission characteristic and the reference transmission characteristic is above a threshold value.

9. The apparatus (100) of any of the preceding claims, wherein the object (102) comprises at least one tablet and the characteristic variable is a mass and / or moisture of the at least one tablet.

10. A method (300) for determining a variable characteristic for an object (102), comprising:

    generating (310) an input signal for a high-frequency filter;
    producing (320) an interaction between the object and an electromagnetic stray field of the high-frequency filter caused by the input signal;
    determining (330), from the input signal and an output signal of the high-frequency filter, a transmission characteristic of the high-frequency filter as a measure for the characteristic variable,
    **characterized in that**
    the input signal (117; 417) is generated by mixing a baseband input signal (113) with a carrier signal (115) in the pass band of the high-frequency filter,

the baseband input signal (113) is generated with a signal bandwidth of at least 10 MHz,

the output signal (121) is mixed with the carrier signal (115) to generate a baseband output signal (137), and

the transmission characteristic is determined from the baseband input signal (113) and the baseband output signal (137).

**11.** The method (300) of claim 10, wherein producing (320) the interaction comprises a movement of the object along the surface of the high-frequency filter.

## Revendications

**1.** Appareil (100) pour déterminer une caractéristique variable d'un objet (102), comprenant :

une unité de génération de signal (110) configurée pour générer un signal d'entrée (113 ; 117);

un filtre haute fréquence (120) avec une entrée pour le signal d'entrée (113 ; 117;), une sortie pour un signal de sortie (121 ; 137), et avec une région (122) pour une interaction entre un champ de dispersion électromagnétique du filtre haute fréquence (120) et l'objet (102) ; et

une unité d'évaluation de signal (130) configurée pour utiliser le signal d'entrée (113 ; 117) et le signal de sortie (121 ; 137) du filtre haute fréquence pour déterminer une caractéristique de transmission du filtre haute fréquence comme mesure de la taille caractéristique de l'objet (102),

**caractérisé par le fait que**

l'unité de génération de signal (110) est configurée pour générer le signal d'entrée (117) en mélangeant un signal d'entrée en bande de base (113) avec un signal porteur (115) dans la bande passante du filtre haute fréquence (120),

l'unité de génération de signal (110) est configurée pour générer le signal d'entrée en bande de base (113) avec une largeur de bande de signal d'au moins 10 MHz,

et

l'unité d'évaluation de signal (130) est configurée pour générer un signal de sortie en bande de base (137) en mélangeant le signal de sortie (121) avec le signal porteur (115) et pour déterminer la caractéristique de transmission sur la base du signal d'entrée en bande de base (113) et du signal de sortie en bande de base (137).

**2.** Appareil (100) selon la revendication 1, comprenant en outre :

un dispositif de transport (140) configuré pour déplacer l'objet (102) le long d'une surface (122) du filtre haute fréquence.

**3.** Appareil (100) selon la revendication 2, dans lequel le dispositif de transport (140) est configuré pour déplacer l'objet (102) le long de la surface d'une manière en contact de surface (122).

**4.** Appareil (100) selon l'une des revendications précédentes dans lequel l'unité d'évaluation de signal (130) est configurée pour établir la caractéristique de transmission en déterminant une différence de phase entre le signal d'entrée (113 ; 117) et le signal de sortie (121 ; 137).

**5.** Appareil (100) selon l'une des revendications précédentes, dans lequel l'unité de génération de signal (110) est configurée pour fournir le signal d'entrée (117) avec une fréquence porteuse dans une gamme de fréquences allant de 300 MHz à 300 GHz.

**6.** Appareil (100) selon l'une des revendications précédentes, dans lequel l'unité de génération de signal (110) est configurée pour pondérer le signal d'entrée en bande de base (113) au moyen d'une fonction fenêtre, en particulier une fonction fenêtre Tuckey.

**7.** Appareil (100) selon l'une des revendications précédentes, dans lequel l'unité d'évaluation de signal (130) est configurée pour déterminer la caractéristique de transmission pour une gamme de fréquences comprenant une pluralité de fréquences.

**8.** Appareil (100) selon l'une des revendications précédentes, dans lequel l'unité d'évaluation de signal (130) est configurée pour comparer la caractéristique de transmission avec une caractéristique de transmission de référence prédéterminée pour l'objet (102) et pour émettre un message d'erreur si une différence entre la caractéristique de transmission et la caractéristique de transmission de référence est supérieure à une valeur-seuil.

9. Appareil (100) selon l'une des revendications précédentes, dans lequel l'objet (102) comprend au moins un comprimé et la variable caractéristique est une masse et/ou une humidité du au moins un comprimé.

10. Procédé (300) pour déterminer une variable qui est caractéristique d'un objet (102), comprenant le fait de :

générer (310) un signal d'entrée pour un filtre haute fréquence ;
produire (320) une interaction entre l'objet et un champ de dispersion électromagnétique du filtre haute fréquence provoqué par le signal d'entrée ;
déterminer (330), à partir du signal d'entrée et d'un signal de sortie du filtre haute fréquence, une caractéristique de transmission du filtre haute fréquence en tant que mesure de la taille caractéristique,
**caractérisé par le fait que**
le signal d'entrée (117 ; 417) est généré en mélangeant un signal d'entrée en bande de base (113) avec un signal porteur (115) dans la bande passante du filtre haute fréquence d,
le signal d'entrée en bande de base (113) est généré avec une largeur de bande de signal d'au moins 10 MHz,
le signal de sortie (121) est mélangé avec le signal porteur (115) pour générer un signal de sortie en bande de base, et
la caractéristique de transmission est déterminée à partir du signal d'entrée en bande de base (113) et du signal de sortie en bande de base (137).

11. Procédé (300) selon la revendication 10, dans lequel produire (320) l'interaction comprend le déplacement de l'objet le long de la surface du filtre haute fréquence.

Fig. 1

EP 3 112 824 B1

FN(ω)

ωm-Δω/2    ωm    ω    ωm+Δω/2

Fig. 2a

| H(f) |

224    220    210    222

fu    fo    f

Fig. 2b

300

```
      ┌──────────────┐
      │     310      │
      └──────────────┘
             │
             ▼
      ┌──────────────┐
      │     320      │
      └──────────────┘
             │
             ▼
      ┌──────────────┐
      │     330      │
      └──────────────┘
             │
             ▼
```

Fig. 3

Fig. 4a

Fig. 4b

510

Fig. 5

300

610

620

630

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4223751 A
- US 5602485 A
- DE OS2939406 A
- US 6163158 A